Europäisches Patentamt

European Patent Office

Office européen des brevets

(19)

(11) **EP 1 323 377 A2**

(12) **EUROPEAN PATENT APPLICATION**

(43) Date of publication:
**02.07.2003 Bulletin 2003/27**

(51) Int Cl.⁷: **A61B 5/16**, A61B 5/11

(21) Application number: **02445194.0**

(22) Date of filing: **27.12.2002**

(84) Designated Contracting States:
**AT BE BG CH CY CZ DE DK EE ES FI FR GB GR
IE IT LI LU MC NL PT SE SI SK TR**
Designated Extension States:
**AL LT LV MK RO**

(30) Priority: **28.12.2001 SE 0104470**

(71) Applicant: **Petter Knagenhjelm
435 43 Pixbo (SE)**

(72) Inventor: **Petter Knagenhjelm
435 43 Pixbo (SE)**

(74) Representative: **Westman, Per Börje Ingemar et al
Ström & Gulliksson IP AB,
Sjöporten 4
417 64 Göteborg (SE)**

(54) **Pattern analysis system and method**

(57) The present invention relates to a computerized comprising: an interface arrangement for interfacing a data source, said data source delivering data related to head motion of a person under influence of drugs, a memory arrangement for storing said data, a processor for processing said data, an artificial neural network (ANN) using said processor, means for collecting a second set of data from said person, said data being collected under influence of a drugs, means for calculating one or several parameters distinctive of various features of said person, and means for feeding said parameter values to said ANN trained to recognize said various features, comprising psychological syndromes.

Figure 2

## Description

FIELD OF THE INVENTION

The present invention relates to an analysis method and a computerized system for detection and prediction of pattern in a person's data. The resulting pattern can be used to characterize the person data and with increased knowledge, the efficiency in predictions, especially but not exclusively, in the medical therapy.

BACKGROUND OF THE INVENTION

Patients who suffer from some psychological syndromes have different movement patterns than a group of people without psychological syndromes. Patient movements can be measured together with other patient data. If the patient's psychological syndrome can be characterized by measured patient data and the use of an objective analysis method it would be possible to observe the effect of the medical therapy. Such analysis method would be very useful.

US 5,810,747 describes an interactive intervention training system used for monitoring a patient suffering from neurological disorders of movement or a subject seeking to improve skill performance and assisting their training. A patient (or trainee) station is used in interactive training. The patient (or trainee) station includes a computer. A supervisor station is used by, for example, a medical or other professional. The patient (or trainee) station and the supervisor station can communicate with each other, for example, over the Internet or over a LAN. The patient (or trainee) station may be located remotely or locally with respect to the supervisor station. Sensors collect physiologic information and physical information from the patient or subject while the patient or subject is undergoing training. This information is provided to the supervisor station. It may be summarized and displayed to the patient/subject and/or the supervisor. The patient/subject and the supervisor can communicate with each other, for example, via video, in real time. An expert system and neural network determine a goal to be achieved during training. There may be more than one patient (or trainee) station, thus allowing the supervisor to supervise a number of patients/subjects concurrently.

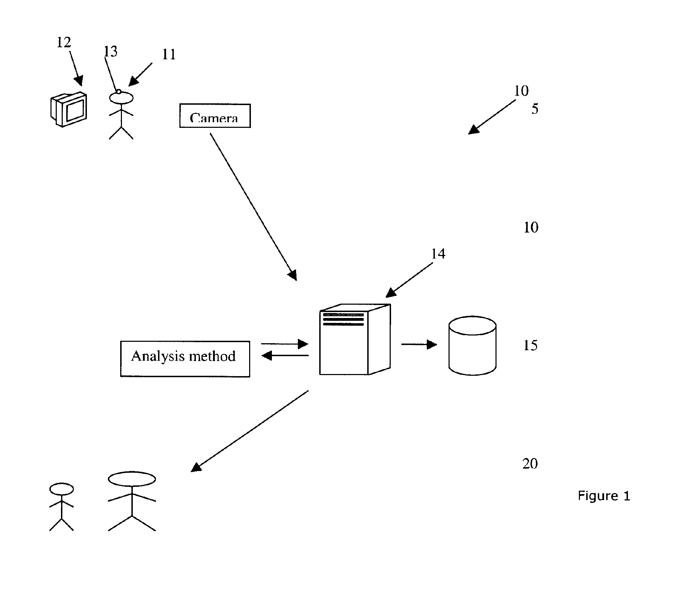

Another known technique is illustrated in Fig. 1; OPTAx, delivered by OPTAx SYSTEMS, Inc. Continuous Performance Test (CPT) is a 15 min test to measure inattention and impulsivity. The patient 11 executes the test itself on a computer 12, while the patient's head motions are measured with a camera. The camera finds the head position by tracking a marker 13, which is fitted on the patients head. Motion data and CPT data are sent to a central system 14 after finalizing the entire test. Test results are calculated on the central system. The results are compiled to a report, which is sent back to a physician. The physician uses the report as one instrument to treat the patient with for example medicines.

In *"Proc. of the 7th IEEE Int. Conf. On Image Processing, 10-13 Sept. 2000, Vol. 2, P. 435-438"* a number of new techniques to enhance the performance of a video analyses system, free from motion markers and complicated setup procedures are described. The system is used for purposes of quantitatively identifying **gait** abnormalities in static human posture analyses. Visual features are determined from still frame images out of the entire walking sequence. The features are used as a guide to train a neural network, in an attempt to assisting clinicians in demagnetizing patients with neurological disorders.

"Real Time Imaging, Vol. 5, No. 4 (Aug. 1999) p. 253-269", describes the application of digital image processing and pattern recognition techniques to assist in diagnosing neurological disorders. In medical practices the posture and movement of human subject through his/her gait cycle contains information that is used by experienced clinician to determine the mental health of a patient. This is achieved by processing, extracting and classifying joint angle information from images of a human subject's **gait.** Joint angles and swing distances obtained from normal and patient subjects are used in training and verifying classifications using feed-forward neural network ad a fuzzy clustering algorithm.

In US 6,090,044, a system for diagnosing medical conditions, such as low back pain (LBP), is provided, whereby a neural network is trained by presentation of large amounts of clinical data and diagnostic outcomes. Following training, the system is able to produce the diagnosis from the clinical data. While the present invention may be useful in diagnosing LBP in one embodiment, other applications of the present invention, both in the medical field and in other fields, are also envisioned. This intelligent diagnostic system is less expensive and more accurate than conventional diagnostic methods, and has the unique capability to improve its accuracy over time as more data is analyzed.

According to WO0064347, a pattern is determined of the neck movement of a subject. The head/body movement of the subject is recorded with markers placed on the shoulders and on the head and thus moving with the subject. The locus curve of each marker in three-dimensional space is then determined in dependence on the time and it is stored as a data set. The neck movement is isolated from the head and torso movements by determining the difference between the average of the two locus curves that represent the shoulder movements and the locus curve representing the head movement. The pattern of movement established on the cranio-corpo-graphy is evaluated and analyzed using a data-processing device. The method is particularly suitable for determining the presence and the severity of an injury to the cervical spine as a result of whiplash caused by a traffic accident

## SUMMARY OF THE INVENTION

It is an object of the preferred embodiment of the present invention to provide a computer system and an analysis method, which distinguishes a personal characteristic, such as a disorder, preferably disorders related to some special type of psychological syndromes. Amongst others, the advantages of the preferred embodiment of the invention involve fast, accurate and classified decision and automatic storage of relevant data. The results involve providing correct amount of drugs more accurately, allow finding/predicting subgroups of patients that are especially responsive to medication and so on.

For these reasons a computerized system is provided, comprising: an interface arrangement for interfacing a data source, said data source delivering data related to motion of a person, a memory arrangement for storing said data, a processor for processing said data, an artificial neural network (ANN) using said processor, means for collecting a second set of data from said person, means for calculating one or several parameters distinctive of various features of said person, means for feeding said parameter values to said ANN trained to recognize said various features. Most preferably, said features comprise psychological syndromes, however, other analysis may also be conducted. In one embodiment, the ANN is trained with data collected from one or several persons being under influence of drugs. Preferably, the ANN comprises a number of nodes representing sets of training data. The system may comprise means for use of Linear Predictive Coding (LPC) to analyze said parameter values fed to said ANN. According to one embodiment, the data source is a camera. Most preferably, the ANN is a Kohonen type ANN.

The invention also relates to a method for the detection of a characteristic of a person employing an artificial neural network (ANN) in which motion data are analyzed, comprising: measuring motion data on said person; collect other measured data from said person; calculating one or several parameters distinctive of various characteristics; feeding said parameter values to an ANN trained to recognize various characteristics, and analyzing said parameter values in the neural network. According to most preferred embodiment, the characteristics include at least one psychological syndrome. The parameters comprise one or several of: the variance of distance, the variance of CPT variables, the residual signal defined as difference between the input signal and a smoothed version of the same, an estimate of immobility duration, and one or more parameters suited to detect periodicity in the one or more of the input signals.

The invention also relates to a method for the detection of patients with psychological syndrome employing an artificial neural network (ANN) in which motion data are analyzed, comprising: measuring motion data on a patient; collect other measured data from the patient; calculating one or several parameters distinctive of various psychological syndromes; feeding said parameter values to an ANN trained to recognize various psychological syndromes, and analyzing said parameter values in the neural network. The parameters comprise one or several of: the variance of distance, the variance of CPT variables, the residual signal defined as difference between the input signal and a smoothed version of the same, an estimate of immobility duration, and one or more parameters suited to detect periodicity in the one or more of the input signals. Preferably, the ANN is trained with data collected from patients being under influence of drugs. According to one embodiment, the ANN comprises a number of nodes representing sets of training data. Most preferably, the ANN is a Kohonen-map type ANN. The method also comprises the use of linear predictive coding (LPC) to analyze the parameter values fed to the ANN. The parameters are used for optimal correlation between parameter distance and conceptual distance. Preferably, the psychological syndrome is ADHD.

Further objects of the invention will be evident from the following short description of the invention, the attached drawings illustrating a preferred embodiment, the detailed description thereof, and the appended claims.

## DESCRIPTION OF THE DRAWING

The invention will be described in the following with reference to attached exemplary drawings, in which:

Figure 1 is a schematic illustration of a system according to the prior art,

Figure 2 is a schematic illustration of a system according to the present invention,

Figure 3 is a block diagram over the system of the invention,

Figure 4 is a schematic illustration of a vector, and

Figure 5 is a schematic illustration of a map response.

## DETAILED DESCRIPTION OF THE EMBODIMENTS

To simplify the description of the present invention, following definitions are used; the definitions are based on a system for patient analyses; however, the invention is not limited to such a system:

*Model*

To distinguish between different signal patterns, a model is used to characterize typical qualities and features of the patient data. The model parameters are chosen with the aim to be as distinct, unambiguous and informative as possible. The set of parameters shall reflect the typical signal patterns.

In addition, to be sensitive to psychological syndrome characteristics, it is important that the parameters shall be insensitive to features irrelevant to the task.

*EventDistanceLimit:*

Minimum distance (eucledian) traveled before it is considered o be a movement.

For example: EventDistanceLimit = 1mm

*Microevent:*

From any point on the movment trajectory, a Microevent is said to occur when the first following point along the trajectoria is reached, where the Eucledian distance between the two points exceeds EventDistanceLim.

*Feature vector*

The values of the model parameters (figure 4) are compiled to form a vector, below named the feature vector. For each subset of patient data, the values of the feature vector are extracted.

Prior to the extraction of parameter values, the signal mean is separate for some signals. The mean will vary with patients and/or hardware and may not contain useful information. The mean is therefore removed in those cases. Each $k$-dimensional feature vector can be regarded as one point in a $k$-dimensional signal-space.

*Training*

An Artificial Neural Network (ANN) is iteratively trained to organize groups or clusters of feature vectors with similar properties. The self-organizing process, known as Self-Organizing Feature Map (SOFM), for example as described in *T. Kohonen "Phonetic typewriter for Finnish and Japanese",* has shown great capability of performing this task.

The number of clusters is defined prior to the training and is determined by the required resolution of the ANN. The training is initiated by a set of (for example M) clusters, randomly positioned in the $k$-dimensional signal-space.

Compiling the feature vectors from a large number of patients forms the database used for training. During the training, each input feature vector is compared to each cluster to find the one with best resemblance to the input vector. This cluster is voted winner, and is adjusted towards the input vector. In addition, all other clusters within a neighborhood to the winner in another domain, the so-called map-space, are adjusted towards the input vector. The map-space is usually of low dimension containing a node for each cluster in the signal-space. The nodes are arranged in hexagonal or a square lattice, and the Euclidean distance between them defines their internal relation. A node's neighborhood is usually defined by a neighborhood function and contains the set all nodes in the beginning of the training whereas only a few (or none) are considered neighbors at the end. The further away a node is to the winner in the map-space, the less the corresponding cluster in the signal-space is adjusted towards the input vector. Thus, all adjustments are done in the signal-space, while the rules of adjustments are defined in the map-space.

The training time is predetermined and an annealing function is used to "freeze" down the system causing only small adjustments at the end of the training. The neighborhood function creates correlation between the signal-space distance and the map-space distance allowing classification to be performed in the (low dimensional) map-space, rather than in the more complicated signal-space.

The method described above is known as "unsupervised learning", i.e. there is no need to use classified data in the training procedure described above.

When the ANN is readily trained, the clusters will represent features of the input signal including normal and various types of psychological syndrome characteristics.

The response (output) of the ANN is proportional to the signal distance between the input signal and all the clusters. See figure 5. Often this output is of less interest in the case of classification. The output is instead used to find the node with best resemblance to a classified input. This is known as the labeling phase in the design of the ANN. Features with know qualities are presented for the ANN, the output is observed and the node giving the highest output is labeled with the presented feature. The actual output thereafter is the label rather than the response value.

The set of clusters are now stored and can then be used in the analysis in runtime mode. Patient data is analyzed exactly the same way as done in the training phase to extract the values of the parameters used in the model i.e. the feature vector. The vector is then presented to the network, which will produce the output label (classification).

Shortly, the present invention is based on the understanding that an analysis of patient data with an Artificial Neural Network (ANN) can successfully be used to distinguish between patients with psychological syndromes and normal patients.

Thus, the present invention provides an Analysis Method (AM) in which patient data, consisting of motion data and other data measured from the patient, is used for calculation of a number of parameters. Patient data are collected from a large number of patients and the data is used to train ANN to teach the system the variation ranges of the parameters. The result from the ANN is obtained as a low-dimensional chart in which each set of patient data is represented by a trajectory. A trajectory for a normal patient looks very different from that for a patient with psychological syndromes.

In particular, the method according to the present invention comprises the following steps:

- Measuring motion data (patients position (or part of body) as a function of time) from a patient;

- Collecting other measured data from the patient;

- Calculating one or several parameters distinctive of patients with psychological syndrome;

- Feeding said parameter values to the artificial neural network trained to recognize psychological syndrome characteristics;

- Analyzing said parameter values in the neural network.

A PREFERRED EMBODIMENT

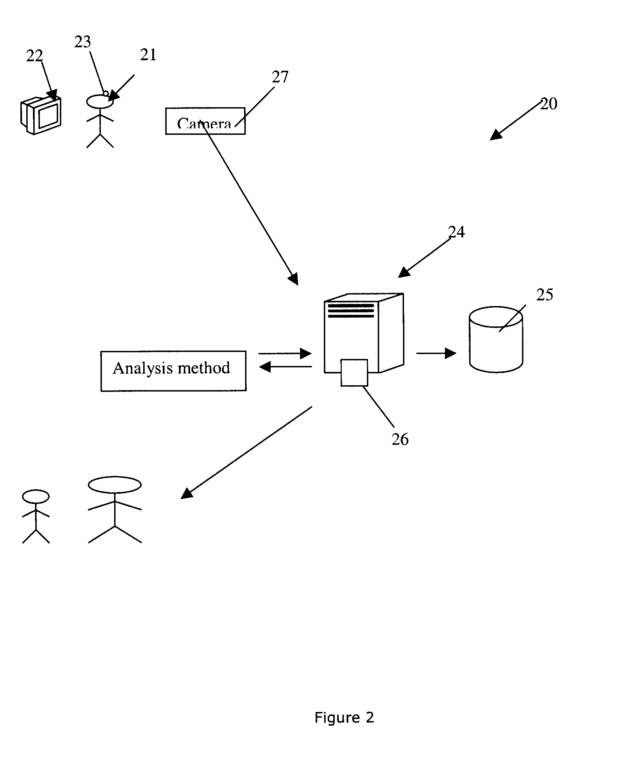

A preferred embodiment of the system 20 according to the invention, is illustrated in figure 2, comprising a computer unit or other training arrangement 22, a central unit 24 connected to a database 25 and comprising AM 26 with an ANN, a camera unit 27 and interface means (not shown) for communication between various parts. The person is provided with a marker 23. The central unit is a conventional computer comprising memory, interface, drivers, storage means, etc. For the purpose of the invention, especially for ADHD, the marker is placed on the head of the person to be analyzed and the motion of the head is analyzed.

The Artificial Neural Network (ANN) 26 is trained with data collected from a large number of patients 21. The data is collected from patients differing in many aspects: sex, age, medical drugs, movement pattern, type of psychological syndrome, etc. The parameter values can be analyzed using a Linear Predictive Coding.

The collected data form a primary database 25. During the training of the artificial neural network, the data is quantified under formation of a small secondary dedicated database, which is used in AM. Thus, according to the present invention, a dedicated secondary database obtained from a primary database comprising data collected from a large number of persons is used in AM. Most specially, the invention offers a new approach as the patients are analyzed and data is collected under the influence of drugs, which is compared to a first collected (system training) data. The patients are analyzed using a reaction test and analyzing a movement patterns, specially movement of the patient's head provided preferably with a detectable arrangement such as a marker. The approach of testing a patient under influence of drugs is thus unique for the invention.

Moreover, the performance of patient can be measured while analyzing the movement pattern. The performance o f the patient can be measured by a switch to be set on and off, giving a reaction time. The performance test can be conducted by providing a patient with very tedious and monotonous test so that the characteristic capacities of a patient are exposed. For example, two different images can be shown in random order, whereby the person to be analyzed must activate the switch for one image and not activate the switch for the other image. The reaction time, number of correct and wrong decisions, and movement pattern are measured during the test. The result of test can be used as a bases for screening report.

The system also comprises a treatment report, which specially arranged for objectively group different types of psychological syndromes, most preferably for ADHD related syndromes. The groups are completely based on the objective measurement data. Some groups react positively to a drug and some in negative way, thus the test under drug influence. Through grouping the patients, it is possible to diagnosis correct treatment. It is also possible to measure the accuracy of a drug dose.

In the system, the ANN comprises a number of nodes representing sets of training data. Each note reflects a state or an incident (feature). Neighboring nodes represent incidents of similar features. In the same way as in training, a feature vector is extracted for each subset of data. The Euclidean distance from the feature vector to each node is calculated. The node in closest proximity to the vector is associated with it. Sequences of incident vectors are followed as sequences of nodes in the artificial neural network. It can be said that a sequence of nodes is the response from the network. Thus, a trajectory in the structure of the network (response) is followed rather than in the parameter space. The fact that the dimension of the network more often is smaller than the parameter space is of advantage, since the calculation thereby is simplified. The response from the network forms the basis for distinguishing between patients with psychological syndrome and normal patients.

The ANN 26 is based on a self-organizing process, known as Self-Organizing Feature Map (SOFM). This type of ANN is preferable to use in this application compare to the other types of ANN, for example MLP neural networks, due to the fact that there is no need for supervised training. The use of unsupervised training makes the training easier to handle large amount of data, less labor intensive and objective. After the training is finalized, the output of the ANN can be labeled with a small amount of classified data. An additional advantage with SOFM ANN is that neighboring nodes in the ANN output represents similar features of the input signal. This implies that the output can be interpreted as a continuum (c.f. soft decision) rather than on-off (c.f. hard decision).

The result from the ANN is presented to competent personal (e.g. physician) by mail, e-mail, through Internet, displayed on a display etc.

Further variations of the present invention are disclosed in the following description of a preferred embodiment.

EXAMPLE 1, APPLICATION ON PATIENT DATA

Equipment: Measuring system from OPTAx, a PC with software based on this analysis method.

Patients: X patients aged from YEARS a to YEARS b, suffering from the psychological syndrome, ADHD, and Y normal patients.

Measurement: The patient was set up with a device for measuring the patient motion during a continuous performance task (CPT). Motion coordinates and data from the CPT were collected. The measuring time was 15 min. Motion data was sampled at 50 Hz, and performance data at 0.5 Hz.

EXAMPLE 2, IMPLEMENTATION

*Data acquisition*

Reference is made to figure 3. Let the input signal(s) be a digitized version of the measured signal(s). Each signal is sampled at certain rate, giving a sequence of samples

$$x_i, i = 0, 1, ..., N$$

*Pre-processing*

To reduce the influence of individual patient variations and to facilitate classification stability, some signals should pass a device to remove the signal mean. Any kind of steep edge high-pass filter can be employed.

*Parameters*

A window is used to calculate parameters on a subset of the data at a time. The window is then slid over the entire measurement. The parameters extracted may be one or more, but not limited to the following.

- The variance of distance, $d_i$ which is defined as

$$\frac{1}{N-1}\sum_{i=1}^{N}(d_i - \bar{d})^2 ,$$

where the distance, d, is defined as the Euclidean distance between two consecutive samples points of motion data, $N$ the number of samples of the complete measurement, and $\bar{d} = 1/N \cdot \sum_{i=1}^{N} d_i$, i.e. the mean movement per sample in meters.

- The variance of CPT variables such as latency defined as

$$\frac{1}{N-1}\sum_{i=1}^{N}(t_i - \bar{t})^2 ,$$

where the latency, $t$, is the delay or reaction time, and $\bar{t} = 1/N \cdot \sum_{i=1}^{N} t_i$, i.e. the mean latency per sample in milliseconds.

- The residual signal defined as difference between the input signal and a smoothed version of the same.

- An estimate of immobility duration.

- One or more parameters suited to detect periodicity in the one or more of the input signals.

*Feature Map geometry and definitions*

Let the M k-dimensional map nodes be denoted

$$m_i, i = 0, \Lambda , M\text{-}1.$$

Most often, the nodes are arranged in a square (2-dimensional) grid. The distance between two map nodes $i$ and $j$, is denoted $D_{i,j}$ and defined as the squared Euclidean distance ($L^2$ norm) between them in the map-space.

$$D_{i,j} = L^2(m_i, m_j).$$

This measure is used in the neighborhood function.

Let the input feature vector, representing sample $x_n$ be denoted $y_n$. Furthermore, let the map response in node $i$ for feature $n$, $S_{i,n}$ be defined as:

$$S_{i,n} = e^{-(d_{i,n}^2/k)}$$

where the signal-space distance $d_{i,n}^2$ is defined as

$$d_{i,n}^2 = \sum_{l=1}^{k} w_l (y_l^n - m_l^i)^2$$

and $w_i$ is some suitable weight function.

*Annealing function*

The task of the annealing function is to obtain equilibrium at the end of the training. The principle is that large adjustments are allowed in the beginning of the training whereas only small (or zero) adjustments are allowed at the end. How the decrease incorporated is not critical. Linear, exponential, and even pulsating [3] decay schedules are proposes in the literature.

*Initialization*

Traditionally, all data driven clustering schemes, including ANNs, employ random positioning of clusters in the signal-space, by assigning (small) random numbers to the parameters. The actual values are not important as long as they are not identical. The ordering of the clusters is also at random.

*Training*

The iterative algorithm adjust all clusters after each input feature vector, $y_n$ , presented. The direction of the adjustment is towards $y_n$, and how much is determined partly by the annealing function, partly by the neighborhood function. The adjustment formulae for cluster $\mathbf{m}_i$ at time instant $t$+1 is:

$$m_i(t+1) = m_i(t) + \gamma_i(t) \cdot (y_n - m_i(t)) , \ i=0, \Lambda \ M-1,$$

where

$$\gamma_k(t) = f(t) \cdot g(t),$$

and $f(t)$ is the annealing function and $g(t)$ is the neighborhood function. Various suitable functions are discussed in *P.Knagenhjelm, "A recursive design method for Robust Vector Quantization"*.

Other parameters used can be, but need not to be limited to:

■  Microevents

The number of position changes greater than *EventDistanceLim.*
■  Immobility duration
  The average time between *Microevents.*
■  Temporal scaling
  Measures the distribution of Immobility duration.
■  Event Distance
  Euclidian distance sampled at *Microevents.*
■  Euclidian Distance
  Euclidian distance sampled at system sampling rate.
■  Area
  Total area (in mm2) covered during the test period.
■  AreaTrend
  A measure of how the covered area varies over test time. The area is measured in three or more sub-intervals. The values are used to fit a curve describing the area evolution. The curve fit may be, for example, polynomial or exponential.
■  Fractal
  A measure of trajectoria complexity.
■  Latency
  Reaction time, i.e. time between target presentation and response.
■  Commission Latency
  As above, but measured at commission errors, i.e. at button presses without target present.
■  LatencyVariation
  Standard deviation of Latency.
■  Coefficient of Variation
  C.O.V = 100 * LatencyVariation/ Latency
■  Commission Errors
  Measures rate of incorrect (switch) button presses.
■  Omission Errors
  Measures rate of incorrect non-presses.
■  MultiResponse
  Measures multiple responses to a single target.

The invention is not limited to the illustrated and described embodiments. Variations and modifications may occur within the scope of the attached claims.

**Claims**

1.  A computerized system comprising:

  -  an interface arrangement for interfacing a data source, said data source delivering data related to head motion of a person under influence of drugs,

  -  a memory arrangement for storing said data,

- a processor for processing said data,

- an artificial neural network (ANN) using said processor,

- means for collecting a second set of data from said person, said data being collected under influence of a drugs,

- means for calculating one or several parameters distinctive of various features of said person; and

- means for feeding said parameter values to said ANN trained to recognize said various features, comprising psychological syndromes.

2. The computerized system of claim 1, wherein said ANN comprises a number of nodes representing sets of training data.

3. The computerized system of claim 1, comprising means for use of Linear Predictive Coding (LPC) to analyze said parameter values fed to said ANN.

4. The computerized system of claim 1, wherein said data source is a camera.

5. The computerized system of claim 1, wherein said ANN is a Kohonen type ANN.

6. The computerized system of claim 1, being provided with a performance test result.

7. The computerized system of claim 1, wherein said motion relates to motion of head of said patient.

8. A method for the detection of a characteristic of a person by means of a computer, which generates a report placing said person in special category needed a treatment, said computer employing an artificial neural network (ANN) in which motion data are analyzed, comprising:

- measuring motion data from said person;

- collect other measured data from said person, under influence of a drug;

- calculating one or several parameters distinctive of various characteristics;

- feeding said parameter values to an ANN trained to recognize various characteristics, and

- analyzing said parameter values in the neural network.

9. The method of claim 8, wherein said parameters comprise one or several of:

- the variance of distance,

- the variance of CPT variables,

- the residual signal defined as difference between the input signal and a smoothed version of the same,

- an estimate of immobility duration, and

- one or more parameters suited to detect periodicity in the one or more of the input signals.

10. The method of claim 9, wherein said variance of distance, $d$, is defined as:

$$\frac{1}{N-1}\sum_{i=1}^{N}(d_i - \bar{d})^2 \, ,$$

where the distance, d, is defined as the Euclidean distance between two consecutive samples points of motion data, $N$ the number of samples of the complete measurement, and $\bar{d} = 1/N \cdot \Sigma_{i=1}^{N} d_i$, i.e. the mean movement per sample in meters.

11. The method of claim 9, wherein said variance of CPT variables such as latency defined as

$$\frac{1}{N-1}\sum_{i=1}^{N}(t_i - \bar{t})^2 \, ,$$

where the latency, $t$, is the delay or reaction time, and $\bar{t} = 1/N \cdot \Sigma_{i=1}^{N} t_i$, i.e. the mean latency per sample in milliseconds.

Figure 1

22 23 21 27 20 24 25 26

Camera

Analysis method

Figure 2